# EUROPEAN PATENT SPECIFICATION

(11) **EP 3 496 761 B1**
(45) Date of publication and mention of the grant of the patent: **21.10.2020**
(21) Application number: 17761924.4
(22) Date of filing: 07.08.2017
(51) Int. Cl.: A61K 47/61, A61P 27/04, A61P 19/02

(54) **FUNCTIONALIZED HYALURONIC ACID**
FUNKTIONALISIERTE HYALURONSÄURE
ACIDE HYALURONIQUE FONCTIONNALISÉ

(30) Priority: 09.08.2016 IT 201600083975
(43) Date of publication of application: 19.06.2019
(73) Proprietor: Consorzio Interuniversitario Nazionale Per La Scienza E Tecnologia Dei Materiali, 50121 Firenze (IT); Universita' Degli Studi di Firenze, 50121 Firenze (IT); Universita' Degli Studi di Siena, 53100 Siena (IT)
(72) Inventor: MAGNANI, Agnese, 53018 Sovicille (IT); NATIVI, Cristina, 50134 Firenze (IT); LEONE, Gemma, 53100 Siena (IT); LAMPONI, Stefania, 53100 Siena (IT); CONSUMI, Marco, 53100 Siena (IT); FRAGAI, Marco, 50019 Sesto Fiorentino (IT); BALDONESCHI, Veronica, 50019 Sesto Fiorentino (IT); RICHICHI, Barbara, 50019 Sesto Fiorentino (IT); FRANCESCONI, Oscar, 52100 Arezzo (IT)
(74) Representative: Valenza, Silvia
(86) International application number: PCT/IB2017/054811
(87) International publication number: WO 2018/029588

(56) References cited:
- EP-A1- 1 082 963
- WO-A1-2016/016847
- WO-A2-2006/013193
- ATTOLINO E ET AL: "Structure-based approach to nanomolar, water soluble matrix metalloproteinases inhibitors (MMPIs)", EUROPEAN JOURNAL OF MEDICINAL CHEMISTRY, EDITIONS SCIENTIFIQUE ELSEVIER, PARIS, FR, vol. 45, no. 12, 1 December 2010 (2010-12-01), pages 5919-5925, XP027526555, ISSN: 0223-5234 [retrieved on 2010-10-20]

## Description

### FIELD OF THE INVENTION

The present invention relates to the field of hyaluronic acid (HA) derivatives. In particular, it relates to molecules derived from hyaluronic acid by conjugation with small matrix metalloproteinases (MMPs) inhibitor molecules. The conjugates of the invention are useful for medical and cosmetic purposes.

### PRIOR ART

Hyaluronic acid (HA) is a linear polysaccharide consisting of the repetition of the disaccharide composed of D-glucuronic acid (GLCA) and N-acetyl D-glucosamine (GlcNAc).

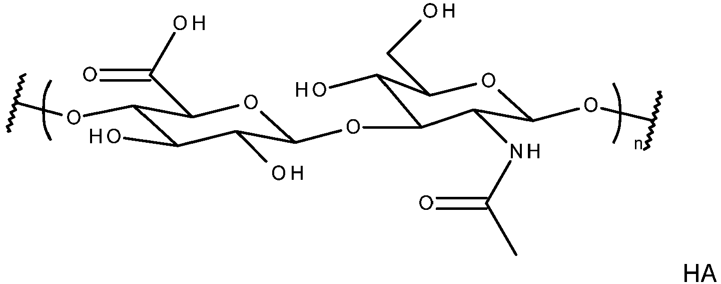

It is part, together with other similar ones, of a group of polysaccharides which is called connective tissue polysaccharides, mucopolysaccharides or glycosaminoglycans. HA is ubiquitous among vertebrates and is abundantly expressed in the extracellular matrix of all tissues, as well as on the cell surface and even within them. Its concentrations range from 0.01-0.1 µg g⁻¹ in blood to 1400-3600 µg g⁻¹ in the synovial fluid. The amount of HA in soft connective tissues ranges from 8.5-18 µg g⁻¹ into the thoracic lymph to 140-338 µg g⁻¹ in the vitreous body. Its peculiar physical and mechanical properties contribute to maintaining the hydration of the tissues (such as in the stroma of the cornea), to their lubrication (bone joints) and to the mediation of the diffusion of solutes through the extracellular space. Despite its simple chemical structure, the HA performs numerous molecular functions that contribute not only to the structural and physiological characterization of the tissues, but also to the modulation of cell behavior during morphogenesis, tissue remodeling and inflammation. The various biological functions of HA are manifested through complex interactions with the matrix components that contribute to the organization and maintenance of the structural integrity of the extracellular and pericellular matrix. In addition, the HA interaction with specific receptors present on the cell surface allows the activation of many cellular functions which contribute to the tissue development and repair, to the modulation of inflammation and the progression of tumor metastasis. The inherent biocompatibility and biodegradability together with the susceptibility to chemical modifications have made HA particularly attractive for the development of biomaterials with promising and broad clinical potential. The particular role of natural lubricant and the excellent properties of retaining water make HA a compound suitable for use in ophthalmic and musculoskeletal products and as filler for the reduction of small wrinkles caused by the local degradation of collagen.

In the eye, HA can be found in the crystalline lens, in lacrimal glands, in the epithelium of the cornea, in the vitreous body and in the conjunctiva. HA has the function of stimulating the migration of epithelial cells and thus has a role in the healing of wounds to the cornea. In fact, HA has been developed both for the recovery of the crystalline lens and for the protection of the corneal endothelium from mechanical trauma during cataract surgeries. It has also been shown how HA is also associated with a protective effect against oxidative damage through the inhibition of free radicals. Finally, HA increases the wettability of the cornea due to its hygroscopic properties, thus being useful within the artificial tears for the treatment of dry eye symptoms. The treatment of the dry eye syndrome may be associated also to the use of therapeutic contact lenses. These, mainly consisting of hydrogels, can be prepared in such a way as to make the controlled release of HA over time possible.

Osteoarthritis, according to the WHO (World Health Organization), affects 25% of adults over the age of 25 years. It is a chronic degenerative and disabling disease severely affecting the quality of life, the possibility of relationship of those affected by it and the healthcare costs of the National Health System. Osteoarthritis leads to the progressive alteration of the anatomical components that form the joints, can affect the vertebrae (the spine) and the joints of the limbs and is characterized by a loss of the articular cartilage, which is replaced by new bone (non-elastic) tissue; this causes pain and limitation of motion. Today, it is well established that in patients with osteoarthritis, there is a marked reduction of the viscoelastic capacities of the synovial fluid. The intra-articular therapy with HA has had great success over the past decade and many patients so treated report feeling a significant benefit to their bone and joint problems. The success of this infiltrative therapy relies mainly on the outstanding viscoelastic properties of hyaluronic acid that replaces the lubrication and cushioning of the synovial fluid.

For cosmetic purposes, the use of HA is a function of its chemical structure which allows a high solvation (formation of hydrogen bonds with many water molecules). Due to this feature, if applied to the skin, it is able to maintain the right level of hydration thereof even in the presence of very low external humidity. This wetting action positively influences the properties of keratin which becomes more flexible and elastic.

Linear HA applied on the skin or injected (in the case of fillers), or infiltrated intra-articularly, as it happens for that naturally present in the dermis and in the joints, is enzymatically degraded in a short time (hours - days). In particular, the HA degradation is caused by the enzyme hyaluronidase. To slow down this physiological process, HA is often chemically modified by crosslinking through treatment with various types of compounds (divinylsulfone, BDDE, epoxides, etc.). In these cases, we speak of cross-linked HA. In this form, the β-1,4 glycosidic bonds, whose cleavage is mediated by hyaluronidases, are less accessible, so the polysaccharide chain degradation process is slowed down. The degradation times in these cases are up to a few weeks. Of course, the greater the cross-linking of HA and the longer will be the time for its complete degradation. The stability at hyaluronidase has also been obtained by functionalizing precise positions of the repeating unit of HA also with satisfactory results, but often accompanied by the alteration of the physical (e.g. rheological) and hydration characteristics of the functionalized product. [T. Segura et al. Biomaterials, 2005, 26, 359].

Among the causes of the diseases mentioned above (ocular, osteo-articular) and also in skin aging there are "inflammatory" states which, although may have among the most diverse etiologies, are all accompanied by the abnormal activation of specific metal-enzymes, matrix metalloproteinases (MMPs). MMPs are a family of 25 Zn peptidases, structurally and functionally very similar which, if hyper-expressed, lead to the uncontrolled degradation of specific protein substrates. Among these MMPs, MMP-1, MMP-9 and MMP-13 attack and cleave the peptide bonds of collagen and gelatin (MMP-9) present in the connective tissue, at an ocular level and in the bones. High concentrations of these MMPs are in fact found in subjects with rheumatoid arthritis (M. Takeshita et al. Arthritis Res Ther 2016, 18, 112, doi: 10.1186/s13075-016-1013-2), osteoarthritis (P. Li et al. Mol Med Rep, 2016, doi: 10.3892/mmr.2016.5419) and eye diseases (N.L. Lanza et al. Ocul Surf, 2016 14, 189, doi: 10.1016/j.jtos.2015.10.004; F. Bian et al. Invest Ophthalmol Vis Sci, 2015, 56, 4908, doi: 10.1167/iovs.15-16631). The "inflammatory" processes, which are accompanied by a high and uncontrolled concentration of MMPs, cause the uncontrolled degradation of protein substrates such as collagen, but also cause a greater degradation of HA.

In this context, in the last thirty years, MMP inhibitors with high affinity towards these metallo-enzymes have been synthesized and tested. Only a few of these, however, have come to clinical trials and with little success. The major limitations of these inhibitors are in fact their poor solubility in water (and hence poor bioavailability) and their poor selectivity. (J.M. Cathcart and J. Cao Front Biosci, 2015, 20, 116) Recently, a group of nanomolar inhibitors has been developed for some MMPs, perfectly soluble in water and therefore with a much better bioavailability compared to those previously known. (C. Nativi et al. in Carbohydrate in Drug Design and Descovery, 2015, pag. 242-252, Eds JJ Barber, F.J. Canada, S. Martin-Santamaria, Royal Society of Chemistry; B. Richichi et al. Chemistry Eur J 2016, 22, 1714; Bartoloni et al. Chem. Eur. J. 2013, 19, 1896 - 1902) These are small molecules with proven inhibitory activity against MMPs involved in the dry eye disease (MMP-2, MMP-9), in inflammatory processes at an osteoarticular level (MMP-3, MMP-12, MMP-13) and in the formation of wrinkles (MMP-1).

EP1082963 discloses conjugates of hyaluronic acid to tryptophan-based MMP inhibitors.

The need to have a HA that is stable to hyaluronidase and maintains the fluidity needed to ensure injectability thereof is therefore clear.

### SUMMARY OF THE INVENTION

The present invention solves the above problems with a derivative of HA functionalized with specific MMP inhibitors, soluble in water.

The object of the present invention is a compound, derivative of HA, of formula (I): 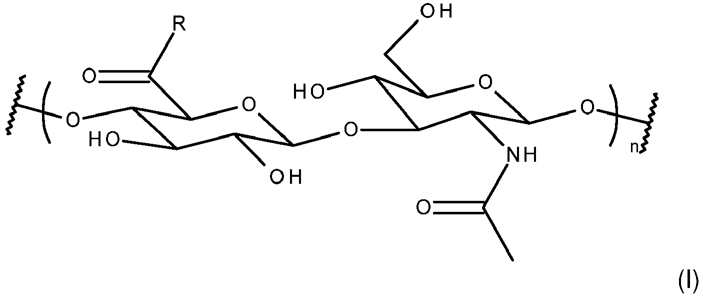 wherein
n is between 450 and 2500;
R is: 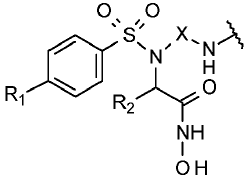 wherein
   R₁ is H, O-Alk, OH, F, Ph, para-C₆H₄-F, OPh, CH₂Ph;
   R₂ is H, CH₂OH, CH₂SH, CH₂CH₂SMe, CH(CH₃)₂, CH(CH₃)OH;
when R₂≠H, the stereocenter has absolute configuration (R);
X is an alkyl chain with a number of atoms of between 3 and 12 optionally containing a triple bond C=C or one or more heteroatoms selected from the group consisting of N, S, O.

This new derivative of HA, while retaining all the non-functionalized HA characteristics (viscosity, injectability, non-toxicity), is more stable to hyaluronidases and is capable of inhibiting specific MMPs hyper-expressed in diseases or blemishes for which the use of HA is already widespread.

Controlling/reducing inflammatory states in which specific MMPs are overexpressed in diseases or blemishes for which the use of HA is already widespread therefore has a double advantage: 1) reducing the inflammatory event (cause of collagen degradation) and 2) slowing down the degradation of both the natural HA and the HA administered through infiltration. Since the HA cleavage produces fragments of the same polysaccharide with a lower molecular weight and which are in turn pro-inflammatory, reducing the degradation of the infiltrated HA would in turn have the beneficial effect of removing a further cause of inflammation in the treated subject. An object of the present invention is also a compound as described above for use as a medicament; in particular, for the treatment of the dry eye disease and of inflammatory processes at an osteoarticular level.

An object of the present invention is also the cosmetic use of a compound as described above, said use in particular for skin blemishes from aging, such as wrinkles.

An object of the present invention is also an injectable cosmetic or pharmaceutical composition, said composition comprising a compound as described above and at least one other pharmaceutically or cosmetically acceptable ingredient.

An object of the present invention is also a method for the preparation of a compound as described above, said method comprising contacting hyaluronic acid with a compound of formula (II) 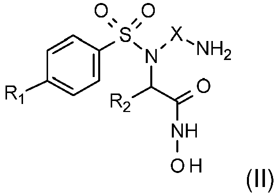 wherein R₁, R₂ and X are as described above
in the presence of suitable coupling reagents for the formation of an amide bond.

### DETAILED DESCRIPTION OF THE INVENTION

Alk means an alkyl residue containing from 1 to 4 atoms of saturated linear or branched C; preferably, Alk is Me, Et, Pr.

For the purposes of the present invention, it is preferable to use a HA having an average molecular weight of 180-200 KDa or 1000 KDa.

A compound according to the invention may exhibit a degree of functionalization of between 1% and 100% of the total repeating units thereof.

The alkyl/heteroalkyl chain X can be linear or branched, saturated or unsaturated; preferably, it is linear.

Preferably, according to the present invention, X is -(CH₂)ₙ-; -(OCH₂CH₂)ₘ-O-, - (NHCH₂CH₂)ₘ-NH-; -(CH₂)ₘ-C≡C-(CH₂)ₘ- wherein n is an integer of between 3 and 12 and m is an integer of between 1 and 3. More preferably, X is -(CH₂)₆-, - OCH₂CH₂O-, -NHCH₂CH₂NH-; -(CH₂)₂-C≡C-(CH₂)₂-

Preferably, R₁ is OMe.

Preferably, R₂ is H or CH₂OH.

Preferably, according to the present invention, R is

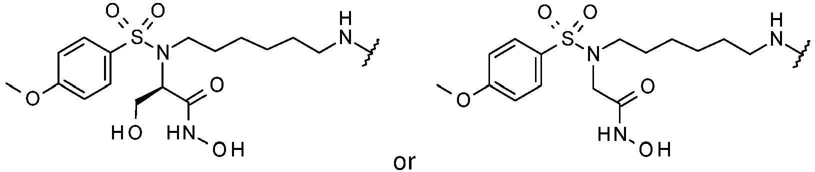

Preferably, the process of preparing a compound according to the invention takes place in homogeneous phase.

Preferably, in the process of preparing a compound according to the invention, the compound of formula (II) is used in a molar ratio of 1:1 with respect to the repeating units of HA (HAᵤᵣ).

Preferably, in the process of preparing a compound according to the invention, the coupling reagents are used in a molar ratio of 10:1 with respect to the repeating units of HA (HAᵤᵣ).

Preferably, in the process of preparing a compound according to the invention, 1-ethyl-3-3(dimethylaminopropyl)carbodiimide hydrochloride (EDC) and N-hydroxy-succinimide (NHS) are used as coupling reagents.

Preferably, the compounds of formula (II) used in the process according to the invention are:

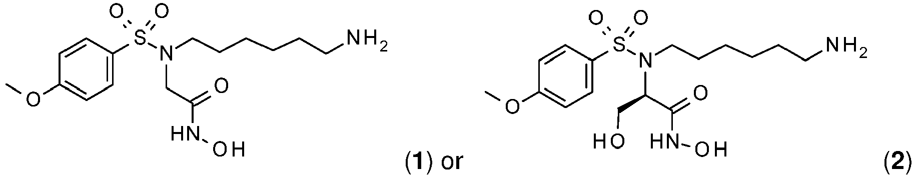

The present invention will be better understood in the light of the following embodiments.

### BRIEF DESCRIPTION OF THE FIGURES

FIG. 1 - IR spectra of: A) HA (grey); b) MMP-HA inhibitor conjugate (black);
FIG. 2 - shows the percentage of vital NIH3T3 fibroblasts 24 hours after contact with the test samples. The results are the average of three experiments repeated in five replicates;
FIG. 3 - Absorbance values measured for MMP-HA and HA inhibitor for a digestion time of 3 hours with the enzyme hyaluronidase;
FIG. 4 - Human primary corneal cell viability after contact with the test samples and subjected to continuous air flow for 0 and 30 minutes. Each sample was assayed in triplicate. Medium: cells not subjected to air flow; PBS: cells in contact with PBS for 20 minutes and then exposed to air flow for 0 and 30 minutes; OPTOyal®A: cells in contact with the tear substitute for 20 minutes and then exposed to air flow for 0 and 30 minutes; MMP-HA inhibitor: cells in contact with hyaluronic acid functionalized with MMP inhibitor for 20 minutes and then exposed to air flow for 0 and 30 minutes.

### EXPERIMENTAL PART

### EXAMPLE 1 - Preparation of inhibitor 2

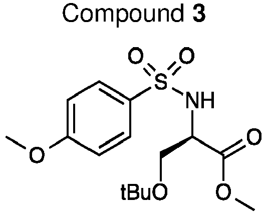

To a solution of H-D-Ser(tBu)-OMe hydrochloride (2 g, 9.45 mmol) in CH₂Cl₂ (20.0 mL) was added triethylamine (3.95 mL, 28.35 mmol) at 0 °C. After 30' at room temperature, the mixture was cooled again to 0 °C and DMAP (0.115 g, 0.94 mmol) and 4-methoxybenzenesulfonyl chloride (1.950 g, 9.45 mmol) were added. After 4 h at room temperature, the reaction mixture was diluted with CH₂Cl₂ (300 mL) and washed with a saturated solution of Na₂SO₄ (3 x 30 mL) and with a saturated solution of NaCl (2 x 30 mL). The organic phase was dried over Na₂SO₄, filtered and concentrated to yield compound **3** as solid yellow (3.210 g, 99%). M.p.: 65-67 °C; ESI-MS 344.14 [M-H]⁻; ¹H NMR (500 MHz, CDCl₃): *δ* 7.79-7.76 (AA' part of an AA'MM' system, *J_{AM}*= 4.7 Hz, 2H), 6.96-6.93 (MM' part of an AA'MM' system, *J_{MA}* = 4.7 Hz, 2H), 5.39-5.38 (bs, 1H), 4.08-4.05 (m, 1H, CHCH₂OtBu), 3.85 (s, 3H, PhOCH₃), 3.68 (A part of an ABX system, *J_{AB}* = 8.9 Hz, *J_{AX}* = 3.2 Hz, CHCH₂OtBu), 3.55 (s, 3H, COOCH₃), 3.52 (B part of an ABX system, *J_{BA}* = 8.9 Hz, *J_{BX}* = 3.6 Hz, CHCH₂OtBu), 1.07 (s, 9H, tBu). ¹³C NMR (125 MHz, CDCl₃): *δ* 170.3 (Cq), 162.9 (Cq), 131.9 (Cq), 129.3 (CH Ar), 114.1 (CH Ar), 73.6 (Cq), 63.0 (CHCH₂OtBu), 56.3 (CHCH₂OtBu), 55.6 (PhOCH₃), 52.4 (COOCH₃), 27.2 (tBu).

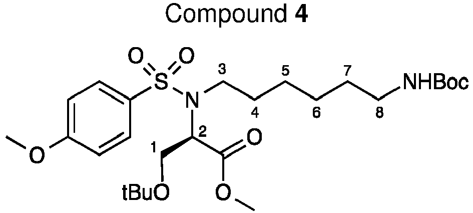

To a solution of **3** (0.700 g, 2.03 mmol), 6-(Boc-amino)-1-hexanol (0.530 g, 2.44 mmol) and PPh₃ (0.640 g, 2.44 mmol) in anhydrous THF (36 mL) was added slowly diisopropyl azodicarboxylate (0.480 mL, 2.44 mmol). After 18 h at room temperature, the reaction mixture was concentrated and the crude product was dispersed in a 8:1 mixture of petroleum ether:ethyl acetate. The dispersion was filtered and the solid was purified by flash chromatography on silica gel (CH₂Cl₂:Acetone 40:1) to yield **4** as glassy white solid (0.922 g, 83%).. [α]_{D}²⁵ +15.17 (c 0.6, CHCl₃); ESI-MS 567.20 [M+Na]⁺; ¹H NMR (500 MHz, CDCl₃): *δ* 7.81-7.78 (AA' part of an AA'MM' system, *J_{AM}*= 9.0 Hz, 2H), 6.97-6.93 (MM' part of an AA'MM' system, *J_{MA}* = 9.0 Hz, 2H), 4.67 (t, *J_{2,1}* = 5.3 Hz, 1H, H-2), 4.56-4.50 (bs, 1H, NH), 3.88 (s, 3H, PhOCH₃), 3.77 (d, *J₁,₂* = 5.4 Hz, 2H, H-1), 3.61 (s, 3H, COOCH₃), 3.30-3.19 (m, 2H, CH₂N), 3.11-3.09 (m, 2H, CH₂N), 1.73-1.56 (m, 2H, CH₂CH₂N), 1.46 (s, 11H, tBu, CH₂CH₂N), 1.34-1.20 (m, 4H, H-5, H-6), 1.14 (s, 9H, Boc). ¹³C NMR (125 MHz, CDCl₃): δ 170.1 (Cq), 162.7 (Cq), 156.0 (Cq), 132.1 (Cq), 129.6 (CH Ar), 113.8 (CH Ar), 61.5 (C-1), 60.2 (C-2), 55.6 (PhOCH₃), 52.0 (COOCH₃), 46.8 (CH₂N), 40.5 (CH₂N), 30.5 (CH₂CH₂N), 30.0 (CH₂CH₂N), 28.4 (tBu), 27.3 (Boc), 26.6-26.5 (C-5, C-6).

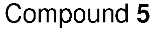

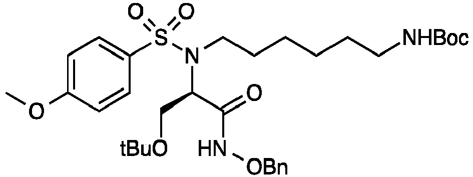

To a solution of **4** (0.700 g, 1.29 mmol) in THF (15 mL) was added an aqueous solution of LiOH·H₂O 1 M (3.87 mL). After 2 h at room temperature, the reaction mixture was acidified with icy acetic acid to pH 5 and concentrated. The crude product (0.676 g) was used in the subsequent reaction. To a crude solution in anhydrous DMF (1.5 mL) was added a solution of TBTU (0.405 g, 1.26 mmol) and NMM (0.153 mL, 1.39 mmol) in anhydrous DMA (1 mL). After 15' at room temperature was added a suspension of BnONH₂·HCl (0.302 g, 1.89 mmol) and NMM (0.228 mL, 2.08 mmol) in anhydrous DMF (1 mL). After 18 h at room temperature, the reaction mixture was concentrated and purified by flash chromatography on silica gel (CH₂Cl₂:acetone 38:1) to yield **5** as a glassy white solid (0.227 g, 58%). ESI-MS 658.23 [M+Na]⁺; ¹H NMR (500 MHz, CDCl₃): *δ* 7.82-7.79 (AA' part of an AA'MM' system, *J_{AM}* = 8.9 Hz, 2H), 7.44-7.35 (m, 5H, Bn), 6.97-6.93 (MM' part of an AA'MM' system, *J_{MA}* = 8.9 Hz, 2H), 4.94-4.89 (m, 2H, CH₂Ph), 4.58-4.49 (bs, 1H, NH), 4.35 (at, 1H, H-2), 3.86 (s, 3H, PhOCH₃), 3.68 (A part of an ABX system, *J_{AB}* = 9.8 Hz, *J_{AX}* = 6.4 Hz, 1H, H-1), 3.57 (B part of an ABX system, *J_{BA}* = 9.8 Hz, *J_{BX}* = 7.7 Hz, 1H, H-1), 3.22-3.12 (m, 2H, CH₂N), 3.11-3.04 (m, 2H, CH₂N), 1.66-1.51 (m, 2H, CH₂CH₂N), 1.45-1.32 (m, 11H, tBu, CH₂CH₂N), 1.32-1.21 (m, 4H, H-5, H-6), 1.02 (s, 9H, Boc). ¹³C NMR (125 MHz, CDCl₃): *δ* 167.4 (Cq), 162.9 (Cq), 156.0 (Cq), 135.2 (Cq), 129.7 (CH Ar), 129.1 (CH Ar), 128.7 (CH Ar), 128.6 (CH Ar), 113.9 (CH Ar), 59.0 (C-1), 58.4 (C-2), 55.7 (PhOCH₃), 45.5 (CH₂N), 40.4 (CH₂N), 30.0 (CH₂CH₂N), 29.9 (CH₂CH₂N), 28.5 (tBu), 27.4 (Boc), 26.5-26.2 (C-5, C-6). 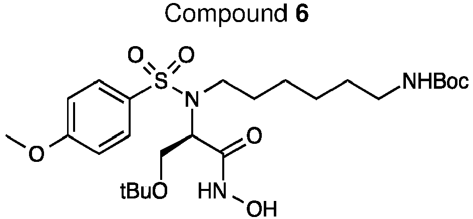 To a solution of **5** (0.105 g, 0.165 mmol) in THF:H₂O 70:1 (6 mL) was added 10% to 50% Pd/C in H₂O (0.070 g, 0.03 mmol). After 10 vacuum/H₂ cycles, the mixture was left at room temperature for 4 h. After 4 h, the reaction mixture was filtered on cotton to yield **6** as a glassy brown solid (0.080 g, 94%). ESI-MS 544.27 [M-H]⁻; ¹H NMR (500 MHz, CD₃OD): *δ* 7.81-7.80 (AA' part of an AA'MM' system, *J_{AM}*= 8.7 Hz, 2H), 7.08-7.06 (MM' part of an AA'MM' system, *J_{MA}* = 8.7 Hz, 2H), 4.58-4.49 (bs, 1H, NH), 4.33 (at, 1H, H-2), 3.89 (s, 3H, PhOCH₃), 3.69 (at, 1H, H-1), 3.46-3.40 (m, 2H, H-1, H-3), 3.26-3.20 (m, 1H, H-3), 3.03 (t, *J₈,₇* = 6.9 Hz, 2H, H-8), 1.73-1.58 (m, 2H, H-4), 1.45 (as, 11H, tBu, H-7), 1.35-1.23 (m, 4H, H-5, H-6), 1.10 (s, 9H, Boc). ¹³C NMR (125 MHz, CD₃OD): *δ* 167.1 (Cq), 162.2 (Cq), 157.2 (Cq), 131.8 (Cq), 129.3 (CH Ar), 113.8 (CH Ar), 59.6 (C-1), 57.7 (C-2), 54.8 (PhOCH₃), 45.2 (C-3), 39.8 (C-8), 30.2 (C-4), 29.5 (C-7), 27.4 (tBu), 26.2 (Boc), 26.2-26.1 (C-5, C-6).

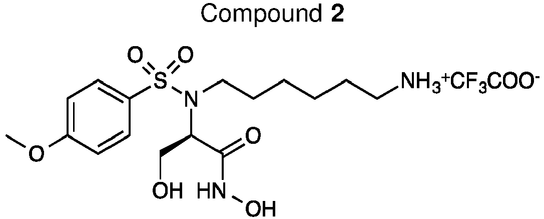

To a solution of **6** (0.070 g, 0.13 mmol) in CH₂Cl₂ (2 mL) was added TFA (0.392 mL, 5.12 mmol). After 18 h at room temperature, the reaction mixture was concentrated to yield the trifluoroacetic salt of **2** as a glassy brown solid (0.064 g, 99%). ESI-MS 390.83 [M+H]⁺; ¹H NMR (500 MHz, CD₃OD): *δ* 7.82-7.80 (AA' part of an AA'MM' system, *J_{AM}*= 8.9 Hz, 2H), 7.09-7.07 (MM' part of an AA'MM' system, *J_{MA}* = 8.9 Hz, 2H), 4.30 (t, *J₂,₁* = 7.1 Hz, 1H, H-2), 3.89 (s, 3H, PhOCH₃), 3.88-3.83 (m, 1H, H-1), 3.60-3.56 (m, 1H, H-1), 3.48-3.42 (m, 1H, H-3), 3.27-3.21 (m, 1H, H-3), 2.93 (t, *J₈,₇* = 7.6 Hz, 2H, H-8), 1.73-1.64 (m, 4H, H-4, H-7), 1.44-1.28 (m, 4H, H-5, H-6). ¹³C NMR (125 MHz, CD₃OD): *δ* 163.3 (Cq), 131.6 (Cq), 129.2 (CH Ar), 113.9 (CH Ar), 59.6 (C-1), 58.5 (C-2), 54.8 (PhOCH₃), 44.9 (C-3), 39.2 (C-8), 29.9 (C-4), 26.9 (C-7), 25.7-25.3 (C-5, C-6).

### EXAMPLE 2 - Functionalization of sodium hyaluronate in homogeneous phase with small MMP inhibitor molecules

0.1 g of hyaluronic acid sodium salt (MW_{UR}= 401) was solubilized in 100 mL distilled water, the solution was mixed under stirring. After 3 minutes of vigorous stirring, the following reagents were added: 1-ethyl-3-3(dimethylaminopropyl)carbodiimide hydrochloride (EDC) (478 mg) and N-hydroxy-succinimide (NHS) (287 mg) and MMP inhibitor molecule **2** (90 mg).

Table 1 shows the molar ratios between the reagents used.

The reaction was left at 25 °C under stirring for 2 h; then, absolute ethanol (400 mL) was added and the solution left at 4 °C O/N. The precipitate was recovered and dissolved in 100 mL of distilled water; the resulting solution was dialyzed for 48 h, then frozen and freeze-dried.

**Table 1. Molar ratios of the polysaccharide/reagents used**

| *Molar ratio Polysaccharide_{UR}*/*MMP inhibitor* | *Molar ratio Polysaccharide_{UR}*/*EDC* | *Molar ratio PolysaccharideU_{R}*/*NHS* |
|---|---|---|
| 1:1 | 1:10 | 1:10 |

### EXAMPLE 3 - Chemical characterization of the MMP-HA inhibitor conjugate obtained from example 2

### IR analysis

The compound obtained according to example 2, purified and dried, was analyzed by infrared spectroscopy using an FT-IR spectrometer (model Thermo Nicolet 5700) equipped with ATR (Attenuated Total Reflectance) accessory with germanium crystal as internal reflection element.

The experimental conditions of acquisition of IR spectra were as follows:
number of scans: 256;
resolution: 4.0 cm⁻¹;
laser energy: 0.46 W;
apodization: Happ-Genzel;
background correction: no.

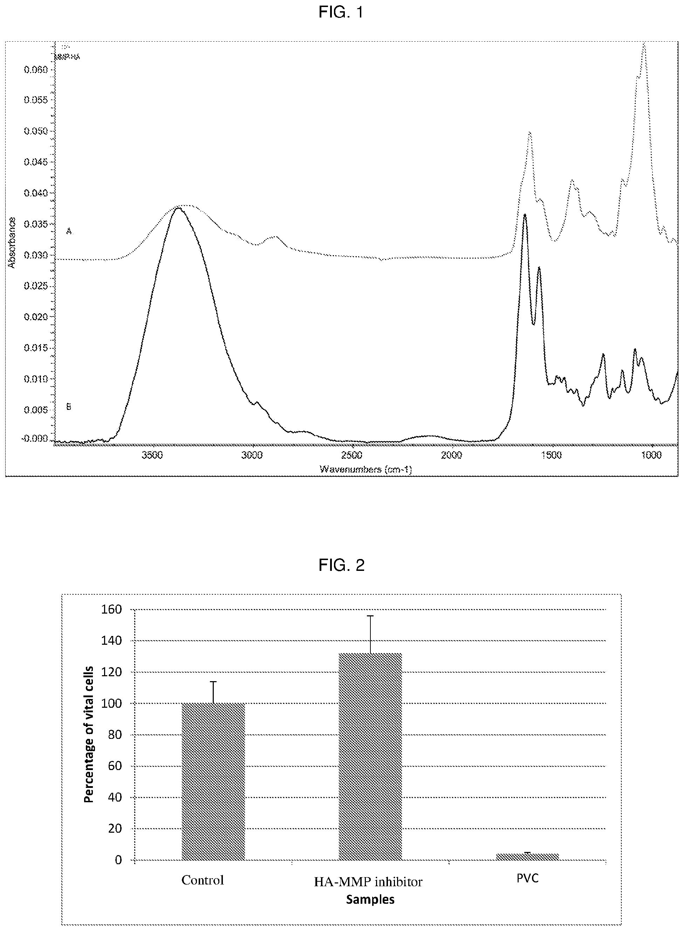

The infrared spectra of HA (gray) and of MMP-HA inhibitor functionalized HA (black) are shown in Figure 1. The main wave numbers observed in the spectra are shown in Table 2 together with their attributions.

**Table 2. Main wave numbers observed in the IR spectra of the native and functionalized HA polysaccharide and their relative attribution.**

| **Wave number (cm⁻¹)** | **Attribution** | **Compound** |
|---|---|---|
| 3320 | Stretching OH (H bonded) | HA; MMP-HA inhibitor |
| 2940-2850 | Stretching CH, CH₂ | HA; MMP-HA inhibitor |
| 1650 | Stretching C=O | HA; MMP-HA inhibitor |
| 1610 | Asymmetric stretching COO⁻ | HA |
| 1550 | Amino NH bending | HA; MMP-HA inhibitor |
| 1420 | OH bending | HA; MMP-HA inhibitor |
| 1403 | Symmetric stretching COO⁻ | HA |
| 1375 | Bending CH, CH₂ | HA; MMP-HA inhibitor |
| 1380-1415 | Stretching S=O | MMP-HA inhibitor |
| 1250, 1180-1153 | Stretching S=O | MMP-HA inhibitor |
| 1150,1075,1040 | Absorption characteristic of polysaccharides | HA; MMP-HA inhibitor |

### EXAMPLE 4 - Biological characterization

### Cytotoxicity test

### Fibroblast cultures

The cytotoxicity of the derivative MMP-HA inhibitor according to the invention was assessed using immortalized mouse fibroblasts (NIH3T3) cultured in polystyrene flasks with DMEM (Dulbecco's Modified Eagle's Medium) supplemented with 10% fetal bovine serum (v/v) (Sigma Chemicals Co., USA), 200 mM L-glutamine (Sigma Chemicals Co., USA) and 5 mg/mL penicillin-streptomycin (Sigma Chemicals Co., USA). The fibroblasts were incubated at 37 °C in atmosphere containing 5% CO₂ up to reaching 80% of cell confluence. The cells were then detached from the flask and separated from each other using a 0.25% v/v solution of sterile trypsin in 0.05% EDTA (Sigma Chemicals Co. USA) and suspended again in complete medium.

### Test execution

Into each well of a 24-well plate for cell culture was placed 1 mL of a suspension containing 8x10⁴ cells/mL. The plate was then incubated for 24 h at 37 °C in 5% CO₂ enriched atmosphere.

After incubation, the culture medium was removed from each well and replaced with 1.0 mL f DMEM containing a 1.3x10⁻⁷ M of the compound to be tested. Each solution was sterilized by filtration (0.22 micron Corning filters). The plates were incubated in an incubator for 24 h. The polystyrene surface of the culture plates was the negative control, while PVC discs stabilized with tin were used as a positive control, as indicated by the ISO standards (ISO-10993-5). After incubation, the cell viability was assessed by testing the incorporation of neutral red, a vital dye that is absorbed only by living cells.

The test consists in adding the neutral red dye (C₁₅H₁₇₄ClN, 3-amino-2-dimethylamino-7-methylphenazine hydrochloride) to the culture medium, which then accumulates in the lysosomes are present in the cytoplasm of living cells. After incubating the samples with the dye, it is extracted from the cells, yielding a solution whose optical density is the number of living cells present. Since the accumulation of the dye is an active process, it will take place mainly in healthy or less damaged cells.

Before running the test, the following solutions were prepared:
- medium with neutral red: 1.0 mL stock solution of neutral red + 99.0 mL culture medium pre-heated to 37 °C;
- stock solution of neutral red (NR): 0.33 g NR in 100 mL of sterile H₂O;
- neutral red extraction solution: 1% (v/v) icy acetic acid + 49% (v/v) H₂O + 50% (v/v) ethanol.

After 24 h incubation, the culture medium was removed and the cells were washed with PBS pre-heated to 37 °C. Finally, 1.0 mL of medium containing the neutral red was added to each well and was incubated for 3 h at 37 °C in humid atmosphere at 5% CO₂. During incubation, the cells were monitored by optical microscope to assess the possible presence of dye crystals that could alter the test results, as they prevented the cells from absorbing the dye. After incubation, the culture medium was removed from the wells and the cells were washed twice with PBS pre-heated to 37 °C. Then, to each well was added 1.0 mL of dye extraction solution and the samples were incubated again at 37 °C in an atmosphere without CO₂ for 20-25 minutes. Finally, the absorbance of the extraction solutions was read at 540 nm by a UV-Vis spectrophotometer (Ultrospec 2000, Pharmacia Biotech, Sweden).

The cytotoxicity test results are shown in Figure 2. For each sample, 5 replicates were repeated for three different experiments. As can be seen from the results shown, the MMP-HA inhibitor compound is not cytotoxic towards NIH3T3 fibroblasts.

### Enzymatic degradation

The enzymatic degradation was assessed based on the method of Ola M. Saad et al., making slight variations to the latter [Saad, or. M., et al. Analytical Biochemistry, 2005, 344, 232-239]. Briefly, in an ammonium acetate buffer at physiological pH, a 1.3x10⁻⁷ M solution of the MMP inhibitor conjugated with hyaluronic acid (MMP-HA inhibitor) was prepared. The solution was then placed in a bath set at 37 °C. To the solution was then added a known amount of hyaluronidase (Sigma) to digest the hyaluronic acid and yield the uronic acid. Hyaluronic acid sodium salt was used as a positive control, the ammonium acetate buffer solution as a negative control.

For each sample was added an excess of enzyme hyaluronidase and the amount of uronic acid, produced as a function of the digestion time, was determined by spectrophotometer, through the carbazole assay [Bitter, T., & Muir, H. M. Analytical Biochemistry, 1962, 4, 330-334 Hedberg, E. L., et al Journal of Controlled Release, 2004, 100, 257-266]. Briefly, 100 µL of the solution from the degradation were added to a solution containing concentrated sulfuric acid and sodium tetraborate decahydrate. The resulting solution was heated to 100 °C for 10 minutes. A solution of 0.125% w/v carbazole in absolute ethanol was then added and the mixture was heated for additional 15 minutes at 100 °C. The final solution absorbance at 530 nm was determined by a UV-visible spectrophotometer (Perkin-Elmer model Lambda 25).

For each sample, 3 replicates were made.

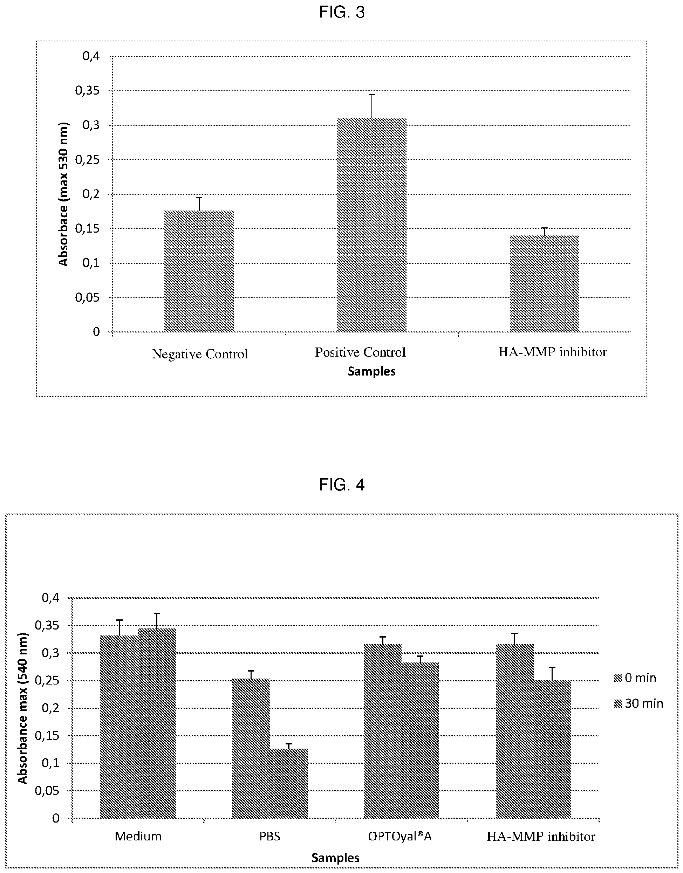

Figure 3 shows the absorbance values for the carbazole assay for a contact time of three h. The susceptibility to enzymatic degradation of the MMP-HA inhibitor compound was compared with sodium hyaluronate (positive control) and with the ammonium acetate buffer solution (negative control).

As shown in Figure 3, the MMP-HA inhibitor has a considerable resistance to hydrolysis, in terms of production of uronic acid, by hyaluronidase. The positive control, i.e. the unmodified hyaluronic acid (HA), is significantly degraded after 3 hours of digestion. This lets assume that over the first hours, digestion is nearly complete, as confirmed by the literature and by the technical specifications provided by the manufacturer of the enzyme.

### EXAMPLE 5 - In vitro assessment of the human corneal cell dehydration prevention Human corneal cell cultures and assessment of their viability after contact with MMP-HA inhibitor

Human corneal cells obtained from donors who had undergone myopia correction surgery were isolated and cultured in vitro at 37 °C in atmosphere containing 5% CO₂ up to achieving confluence in DMEM (Dulbecco's Modified Eagle's Medium) supplemented with 10% (v/v) fetal bovine serum (Sigma Chemicals Co., USA), 200 mm L-glutamine (Sigma Chemicals Co., USA) and 5 mg/mL penicillin-streptomycin (Sigma Chemicals Co., USA). Once 80% confluence was achieved, after removing the culture medium, the cells were washed 3 times with 100 µL PBS (1x) and to each well were added 30 µL (initial solution concentration 0.150 g/100 mL) of the solution of MMP inhibitor molecule conjugated with hyaluronic acid (MMP-HA-inhibitor) in PBS buffered at about pH = 7.2.

PBS buffered at pH = 7.2 was used as a negative control and OPTOyal ®A commercial tear substitute as positive control (SOOFT Italia S.p.A., Fermo, Italy, which consists of an ophthalmic solution with 0.15% sodium hyaluronate and amino acids). The cells in contact with the test samples were then incubated for 20 minutes at 37 °C in atmosphere containing 5% CO₂. After incubation, the solutions being tested were removed, the cells were washed with 100 µL PBS (1x) and subjected to continuous air flow for 0 and 30 minutes at room temperature. Finally, the cells were incubated for 3 hours at 37 °C in atmosphere containing 5% CO₂ in the presence of medium containing the vital dye neutral red. The neutral red assay was performed according to the procedure described above (see the Cytotoxicity test paragraph). Each sample was assayed in triplicate.

As shown in the diagram in Fig. 4, it is seen that the treatment of cells with the commercial tear substitute and the MMP-HA inhibitor molecule reduces the number of viable cells after exposure of the cell monolayer to 30 minutes of continuous air flow with respect to untreated cells (Medium), but significantly less than what happens for those treated with PBS. In addition, the cell viability after 30 minutes of contact with OPTOyal®A is not statistically different than that observed after contact with the MMP-HA inhibitor molecule. This is especially important considering that the commercial product that contains, in addition to hyaluronic acid, also amino acids that contribute to increasing the lubricating effect of hyaluronic acid, proves to have the same effectiveness of the MMP-HA inhibitor molecule.

## Claims

1. A compound, derivative of HA, of formula (I): 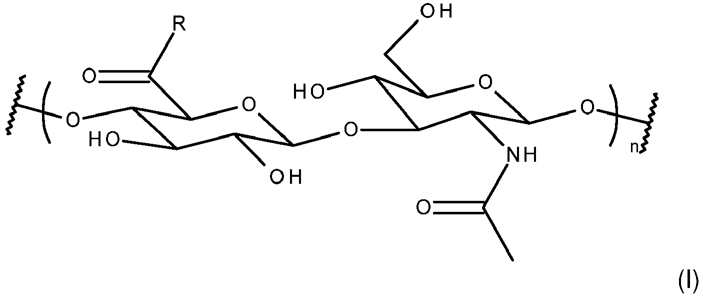 wherein
n is between 450 and 2500;
R is 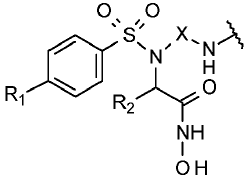 wherein
R₁ is H, O-Alk, OH, F, Ph, para-C₆H₄-F, OPh, CH₂Ph;
R₂ is H, CH₂OH, CH₂SH, CH₂CH₂SMe, CH(CH₃)₂, CH(CH₃)OH;
when R₂≠ H, the stereocenter has absolute configuration (R);
X is an alkyl chain with a number of atoms of between 3 and 12 optionally containing a triple bond C≡C or one or more heteroatoms selected from the group consisting of N, S, O.

2. A compound according to claim 1, wherein X is -(CH₂)ₙ-; -(OCH₂CH₂)ₘ-O-, -(NHCH₂CH₂)m-NH-; -(CH₂)ₘ-C≡C-(CH₂)ₘ- wherein n is an integer of between 3 and 12 and m is an integer of between 1 and 3.

3. A compound according to claim 1 or 2, wherein R₁ is OMe.

4. A compound according to any one of claims 1-3, wherein R₂ is H or CH₂OH.

5. A compound according to any one of claims 1-4, wherein R is

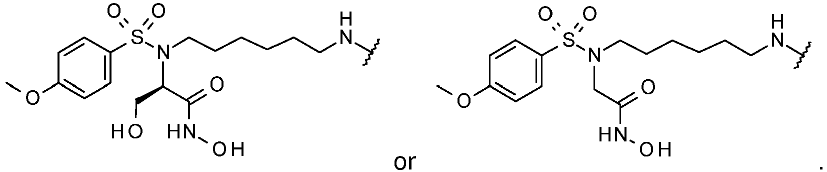

6. A compound according to any one of claims 1-5 for use as a medicament.

7. A compound according to any one of claims 1-5 for use in the treatment of the dry eye disease and of inflammatory processes at an osteoarticular level.

8. Cosmetic use of a compound according to any one of claims 1-5.

9. A pharmaceutical or cosmetic composition, said composition injectable, said composition comprising a compound according to any one of claims 1-5 and at least one other pharmaceutically or cosmetically acceptable ingredient.

10. A method for preparing a compound according to any one of claims 1-5, said method comprising contacting hyaluronic acid with a compound of formula (II) 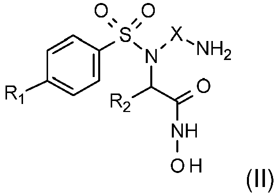 wherein R₁, R₂ and X are as described above

## Patentansprüche

1. Verbindung, die ein Derivat von HA ist, der Formel (I): 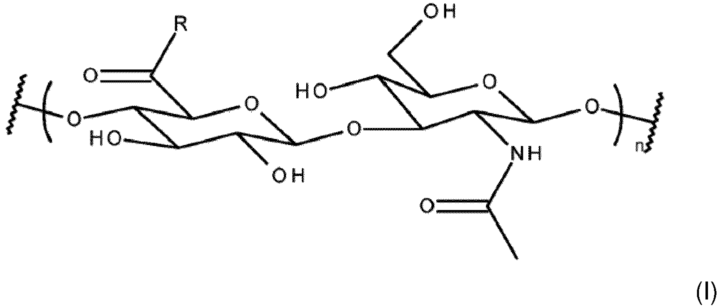 wobei
n zwischen 450 und 2500 liegt;
R 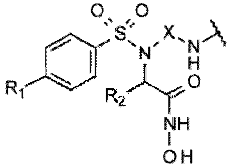 ist,
wobei
R₁ H, O-Alk, OH, F, Ph, para-C₆H₄-F, OPh, CH₂Ph ist;
R₂ H, CH₂OH, CH₂SH, CH₂CH₂SMe, CH(CH₃)₂, CH(CH₃)OH ist;
wenn R₂ ≠ H, das Stereocenter eine absolute Konfiguration (R) aufweist;
X eine Alkylkette mit einer Anzahl von Atomen zwischen 3 und 12 ist, die gegebenenfalls eine Dreifachbindung C≡C oder ein oder mehrere Heteroatome enthält, ausgewählt aus der Gruppe bestehend aus N, S, O.

2. Verbindung gemäß Anspruch 1, wobei X -(CH₂)ₙ-; -(OCH₂CH₂)ₘ- O-, -(NHCH₂CH₂)ₘ-NH-; -(CH₂)ₘ-C≡C-(CH₂)ₘ- ist, wobei n eine ganze Zahl zwischen 3 und 12 ist und m eine ganze Zahl zwischen 1 und 3 ist.

3. Verbindung gemäß Anspruch 1 oder 2, wobei R₁ OMe ist.

4. Verbindung gemäß einem der Ansprüche 1-3, wobei R₂ H oder CH₂OH ist.

5. Verbindung gemäß einem der Ansprüche 1-4, wobei R 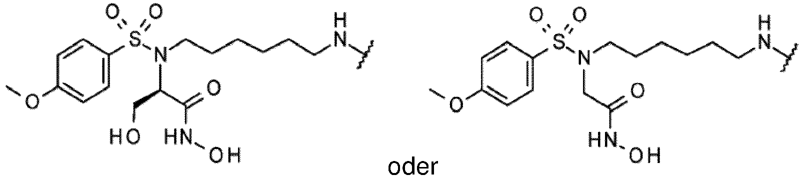 ist.

6. Verbindung gemäß einem der Ansprüche 1-5 zur Verwendung als Medikament.

7. Verbindung gemäß einem der Ansprüche 1-5 zur Verwendung bei der Behandlung des trockenen Augensyndroms und von Entzündungsprozessen auf osteoartikulärer Ebene.

8. Kosmetische Verwendung einer Verbindung gemäß einem der Ansprüche 1-5.

9. Pharmazeutische oder kosmetische Zusammensetzung, wobei die Zusammensetzung injizierbar ist, wobei die Zusammensetzung eine Verbindung gemäß einem der Ansprüche 1-5 und mindestens einen weiteren pharmazeutisch oder kosmetisch annehmbaren Wirkstoff umfasst.

10. Verfahren zur Herstellung einer Verbindung gemäß einem der Ansprüche 1-5, wobei das Verfahren Folgendes umfasst: das In-Kontakt-Bringen von Hyaluronsäure mit einer Verbindung der Formel (II) 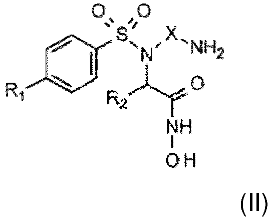 wobei R₁, R₂ und X wie oben beschrieben sind.

## Revendications

1. Composé, dérivé de l'AH, de formule (I) : 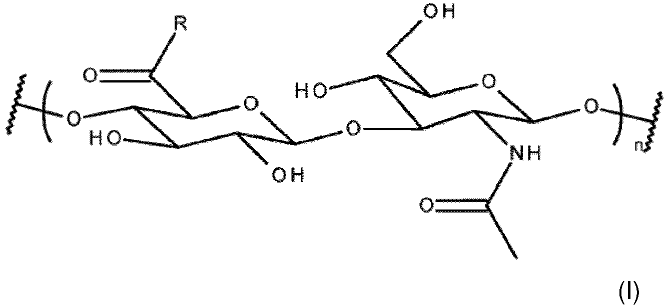 où
n est compris entre 450 et 2500 ;
R est 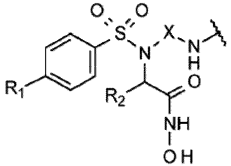 où
R₁ est H, O-Alk, OH, F, Ph, para-C₆H₄-F, OPh, CH₂Ph ;
R₂ est H, CH₂OH, CH₂SH, CH₂CH₂SMe, CH(CH₃)₂, CH(CH₃)OH ;
lorsque R₂ ≠ H, le centre stéréogène a une configuration absolue (R) ;
X est une chaîne alkyle avec un nombre d'atomes compris entre 3 et 12, contenant éventuellement une triple liaison C≡C ou un ou plusieurs hétéroatomes sélectionnés dans le groupe composé de N, S, O.

2. Composé selon la revendication 1, dans lequel X est -(CH₂)ₙ- ; -(OCH₂CH₂)ₘ- O- ; -(NHCH₂CH₂)ₘ-NH- ; -(CH₂)ₘ-C≡C-(CH₂)ₘ- où n est un entier compris entre 3 et 12 et m est un entier compris entre 1 et 3.

3. Composé selon la revendication 1 ou 2, dans lequel R₁ est OMe.

4. Composé selon l'une quelconque des revendications 1 à 3, dans lequel R₂ est H ou CH₂OH.

5. Composé selon l'une quelconque des revendications 1 à 4, dans lequel R est

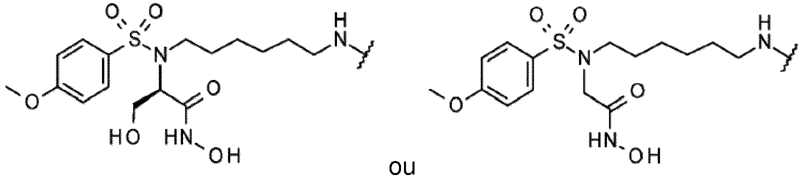

6. Composé selon l'une quelconque des revendications 1 à 5, destiné à une utilisation comme médicament.

7. Composé selon l'une quelconque des revendications 1 à 5, destiné à une utilisation dans le traitement de la kératoconjonctivite sèche ou de processus inflammatoires à un niveau ostéoarticulaire.

8. Utilisation cosmétique d'un composé selon l'une quelconque des revendications 1 à 5.

9. Composition pharmaceutique ou cosmétique, ladite composition étant injectable, ladite composition comprenant un composé selon l'une quelconque des revendications 1 à 5 et au moins un autre ingrédient acceptable sur le plan pharmaceutique ou cosmétique.

10. Procédé de préparation d'un composé selon l'une quelconque des revendications 1 à 5, ledit procédé comprenant la mise en contact d'acide hyaluronique avec un composé de formule (II) 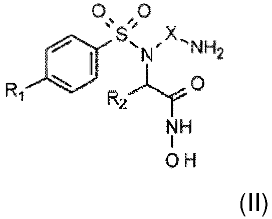 où R₁, R₂ et X sont tels que décrits ci-dessus.
